# EUROPÄISCHE PATENTANMELDUNG

(11) **EP 2 332 514 A1**
(43) Veröffentlichungstag der Anmeldung: **15.06.2011**
(21) Anmeldenummer: 10172275.9
(22) Anmeldetag: 09.08.2010
(51) Int. Cl.: A61K 8/19, A61K 8/34, A61K 8/43, A61Q 11/00

(54) **Antibakterielle Mund- und Zahnpflege- und -Reinigungsmittel mit Silber**

(30) Priorität: 14.12.2009 DE 102009054610
(71) Anmelder: Henkel AG & Co. KGaA, 40589 Düsseldorf (DE)
(72) Erfinder: Adomat, Christel, 40591, Düsseldorf (DE); Adelmann, Barbara, 40597, Düsseldorf (DE); Barth, Adolf Peter, 42781, Haan (DE); Simmering, Rainer, 41515, Grevenbroich (DE)

(57) **Zusammenfassung**

Die antibakterielle Wirkung antimikrobieller Substanzen lässt sich überraschenderweise durch den Einsatz von Silberionen steigern. Entsprechende vorteilhafte Mittel sind insbesondere Mund und- und Zahnpflege- und -reinigungsmittel, die - bezogen auf ihr Gewicht - 0,1 bis 100 ppm Silberionen und 0,001 bis 5 Gew.-% mindestens einer antibakteriellen Verbindung enthalten.

## Beschreibung

Die Erfindung betrifft Zubereitungen zur Mund- und Zahnpflege- und -reinigung, die aufgrund von speziellen Inhaltsstoffen eine ausgezeichnete antibakterielle Wirkung besitzen.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Bakterien bekämpfen und damit Zahnbelag (Plaque), Gingivitis, Paradontitis und Mundgeruch verringern und/oder verhindern. Mundgeruch, auch Halitosis oder "Foetor ex ore" genannt, kann verschiedene Ursachen haben. Bei gesunden Menschen wird Mundgeruch jedoch in den meisten Fällen durch Bakterien im Mund- bzw. Rachenraum hervorgerufen, die Körperzellen oder Nahrungsreste verstoffwechseln, wobei als Abbauprodukte von Proteinen u.a. flüchtige Schwefelverbindungen ("volatile sulfur compounds", VSC) entstehen, die für den üblen Geruch verantwortlich gemacht werden. Diese Schwefelverbindungen sind insbesondere: H₂S = Schwefelwasserstoff (zu etwa 30 %), CH₃-S-H = Methylmercaptan (zu etwa 60 %) und CH₃-S-CH₃ = Dimethylsulfid (zu etwa 10 %).

Die flüchtigen Schwefelverbindungen sind teilweise sehr aggressiv und können das Zahnfleisch und die Mundschleimhaut schädigen. So ist beispielsweise bekannt, daß Schwefelwasserstoff und Methylmercaptan die Durchlässigkeit der Mundschleimhaut erhöhen und so Zahnfleischerkrankungen Vorschub leisten können.

Es hat nicht an Versuchen gefehlt, Bakterien im Mundraum zu bekämpfen, und eine Vielzahl von Agentien wird zu diesem Zweck in Zubereitungen zur Mund- und Zahnpflege und -reinigung eingesetzt. Als antibakterielle Mittel finden beispielsweise Triclosan, Zinnsalze oder Chlorhexidin Verwendung. Der Einsatz dieser Produkte kann jedoch in Einzelfällen mit Nachteilen verbunden sein, die teils geschmacklicher Natur sind, teils ästhetische Gründe haben, da das Produkt und/oder die Zähne durch übermäßigen Gebrauch der Agentien verfärbt werden können.

Es besteht daher nach wie vor das Bedürfnis, Bakterien im Mundraum zu bekämpfen und Wirkstoffe oder Wirkstoffkombinationen dafür bereitzustellen, die frei von den genannten Nachteilen sind.

Die Aufgabe der vorliegenden Erfindung bestand darin, Zubereitungen zur Mund- und Zahnpflege und -reinigung bereitzustellen, die Mundgeruch wirksam bekämpfen und gegen Plaque, Gingivitis und Parodontitis wirksam sind. Dabei sollten die Nachteile des Einsatzes von Triclosan, Zinnsalzen oder Chlorhexidin vermieden, deren Einsatzmenge verringert oder auf natürliche antimikrobielle Stoffe zurückgegriffen werden können.

Es wurde nun gefunden, daß sich bestimmte Inhaltsstoffe zur Lösung des vorstehend genannten Aufgabenkomplexes eignen.

Gegenstand der vorliegenden Erfindung ist in einer ersten Ausführungsform ein Mund- und Zahnpflege- und -reinigungsmittel, enthaltend - bezogen auf sein Gewicht -
a) 0,1 bis 100 ppm Silberionen,
b) 0,001 bis 5 Gew.-% mindestens einer antibakteriellen Verbindung.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Die erfindungsgemäßen Mittel enthalten - bezogen auf das Gewicht des Mittels - als ersten Inhaltsstoff 0,1 bis 100 ppm Silberionen. Vorzugsweise wird/werden diese innerhalb engerer Mengenbereiche eingesetzt, wobei bevorzugte erfindungsgemäße Mund- und - und Zahnpflege-und -reinigungsmittel - bezogen auf ihr Gewicht - - 0,2 bis 75 ppm, vorzugsweise 0,5 - 50 ppm, besonders bevorzugt 0,75 - 25 ppm und insbesondere 1 bis 10 ppm Silberionen enthalten.

Die erfindungsgemäßen Zusammensetzungen enthalten 0,1 bis 100 ppm Silberionen. Der Begriff "ppm" ("Teile von einer Million") steht für die Zahl 10⁻⁶ und wird in der Wissenschaft für den millionsten Teil verwendet, so wie Prozent (%) für den hundertsten Teil steht. "ppm" ist eine relative Maßangabe bedeutet dabei, daß ein Teil auf eine Million Teile enthalten ist. Im Rahmen der vorliegenden Anmeldung bezieht sich ppm immer auf Gewichtsteile, d.h. 1 ppm entspricht 0,0001 Gew.-%.

In anderen Worten enthalten die erfindungsgemäßen Mittel als Inhaltsstoff a) anspruchsgemäß 0,00001 bis 0,01 Gew.-% Silberionen.

Als Silberionenquelle werden in den erfindungsgemäßen Mittel vorzugsweise leicht lösliche Silbersalze eingesetzt. Ganz besonders bevorzugt sind Silbersalze von organischen Säuren. Insbesondere bevorzugte erfindungsgemäße Mund- und Zahnpflege- und reinigungsmittel sind **dadurch gekennzeichnet, daß** sie mindestens ein Silbersalz aus der Gruppe Silbercitrat, Silberlactat, Silberacetat, Silbersulfat, Silbermala, Silbersuccinat, Silbertartrat, Silbermandelat, Silbersalicylat, Silbergluconat enthalten.

Unter diesen Salzen ist wiederum das Silbercitrat (tri-Silbercitrat) AgO₂CCH₂C(OH)(CO₂Ag)CH₂CO₂Ag besonders bevorzugt. Ganz besonders bevorzugte erfindungsgemäße Mund- und Zahnpflege- und -reinigungsmittel enthalten daher - bezogen auf ihr Gewicht- 0,15 bis 150 ppm (1,5 10⁻⁶ bis 0,0015 Gew.-%), vorzugsweise 0,25 bis 125 ppm (2,5 10⁻⁶ bis 0,00125 Gew.-%), besonders bevorzugt 0,5 bis 100 ppm (5 10⁻⁶ bis 0,001 Gew.-%) und insbesondere 1 bis 90 ppm (1 10⁻⁵ bis 0,0009 Gew.-%) Silbercitrat.

Es ist besonders bevorzugt, Silbercitrat als einzige Quelle von Silberionen in die erfindungsgemäßen Mittel zu inkorporieren. Erfindungsgemäße Mund und Zahnpflege- und - reinigungsmittel, die als Silbersalz ausschließlich Silbercitrat enthalten, sind erfindungsgemäß bevorzugt.

Als zweite wesentliche Komponente enthalten die erfindungsgemäßen Mittel 0,001 bis 5 Gew.-% mindestens einer antibakteriellen Verbindung. Erfindungsgemäß bevorzugte Mund- und Zahnpflege- und -reinigungsmittel sind **dadurch gekennzeichnet, daß** sie - bezogen auf ihr Gewicht - 0,0025 bis 2,5 Gew.-%, vorzugsweise 0,005 bis 1 Gew.-%, besonders bevorzugt 0,01 bis 0,5 Gew.-% und insbesondere 0,025 bis 0,1 Gew.-% antibakterielle(n) Wirkstoff(e) enthalten.

In Mund und Zahnpflege- und -reinigungsmitteln können beispielsweise die auch in Lebensmitteln zugelassenen Konservierungsmittel Sorbinsäure (E 200), Kaliumsorbat (E 202), Calciumsorbat (E 203), Benzoesäure (E 210), Natriumbenzoat (E 211), Kaliumbenzoat (E 212), Calciumbenzoat (E 213), Ethyl-4-hydroxybenzoat (E 214), Ethyl-4-hydroxybenzoat, Natriumsalz (E 215), Propyl-4-hydroxybenzoat (E 216), Propyl-4-hydroxybenzoat, Natriumsalz (E 217), Methyl-4-hydroxybenzoat (E 218), Methyl-4-hydroxybenzoat, Natriumsalz (E 219), Schwefeldioxid (schweflige Säure), (E 220), Natriumsulfit (E 221), Natriumhydrogensulfit (E 222), Natriumdisulfit (E 223), Kaliumdisulfit (E 224), Calciumsulfit (E 226), Calciumhydrogensulfit (E 227), Kaliumhydrogensulfit (E 228), Biphenyl (E 230), Orthophenylphenol (2-Biphenylol), (E 231), Natriumorthophenylphenolat (E 232), Nisin (E 234), Natamycin (E 235), Ameisensäure (E 236), Natriumformiat (E 237), Calciumformiat (E 238), Hexamethylentetramin (E 239), Dimethyldicarbonat (E 242), Kaliumnitrit (E 249), Natriumnitrit (E 250), Natriumnitrat (E 251), Kaliumnitrat (E 252), Essigsäure (E 260), Kaliumacetat (E 261), Natriumacetat (E 262), Calciumacetat (E 263), Milchsäure (E 270), Propionsäure (E 280), Natriumpropionat (E 281), Calciumpropionat (E 282), Kaliumpropionat (E 283), Borsäure (E 284), Natriumtetraborat (E 285), Hydroxybernsteinsäure (Äpfelsäure), (E 296), Fumarsäure (E 297), Lysozym (E 1105), eingesetzt werden.

Bevorzugte Stoffe sind ausgewählt aus p-Hydroxybenzoesäuremethyl-, -ethyl- oder propylester, Natriumsorbat, Natriumbenzoat, Bromchlorophen, Triclosan, Phenyl- Salicylsäureester, Biguaniden z. B. Chlorhexidin (1,1'-Hexamethylenbis[5-(4-chlorphenyl)-biguanid), Thymol usw..

Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Besonders bevorzugte antimikrobielle Komponenten sind halogenierte Diphenylether, z.B. 2,4-Dichlor-2'-hydroxydiphenylether, 4,4'-Dichlor-2'-hydroxydiphenylether, 2,4,4'-Tribrom-2'-hydroxydiphenylether und 2,4,4'-Trichlor-2'-hydroxydiphenylether (Triclosan).

In bevorzugten erfindungsgemäßen Mund- und Zahnpflege- und -reinigungsmitteln ist/sind der bzw. die antibakteriellen Wirkstoffe ausgewählt aus p-Hydroxybenzoesäuremethyl-, -ethyl- oder propylester und/oder Bromchlorophen und/oder Triclosan und/oder Phenyl-Salicylsäureester und/oder Biguaniden, vorzugsweise Chlorhexidin.

Aus geschmacklichen Gründen oder aus Gründen des Verzichts auf synthetische Verbindungen können natürliche antimikrobielle Stoffe bevorzugt sein. Deren Wirkung wird durch die erfindungsgemäße Komponente a) ebenfalls synergistisch verstärkt, so dass sie auch in kleinen Dosen hocheffektiv wirken. Besonders bevorzugt sind daher auch erfindungsgemäße Mund- und Zahnpflege- und -reinigungsmittel, bei denen der bzw. die antibakteriellen Wirkstoffe ausgewählt ist/sind aus Thymol und/oder Eugenol und/oder Eucalyptol und/oder Extrakten von Myrrhe und/oder Extrakten von Salbei und/oder Extrakten von Nelke.

Die erfindungsgemäßen Mund- und Zahnpflege- und -reinigungsmittel enthalten weitere Inhaltsstoffe. Bevorzugt ist hierbei der Einsatz von so genannten Feuchthaltemitteln, die bei Zahnpasten das Austrocknen verhindern. Bei so genannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel. Hier sind erfindungsgemäße Mund- und Zahnpflege- und -reinigungsmittel bevorzugt, die - bezogen auf ihr Gewicht - 0,5 bis 60 Gew.-%, vorzugsweise 0,75 bis 55 Gew.-%, besonders bevorzugt 1 bis 50 Gew.-% und insbesondere 2 bis 40 Gew.-% mindestens eines mehrwertigen Alkohols aus der Gruppe Sorbit und/oder Glycerin und/oder 1,2-Propylenglycol.-% oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so daß Mittel, die außer Sorbit keine anderen mehrwertigen Alkohole enthalten, besonders bevorzugt sind.

Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.

Erfindungsgemäße Mund- und Zahnpflege- und -reinigungsmittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise in Mengen von 0,01 bis 5 Gew.%, vorzugsweise von 0,02 bis 2,5 Gew.% und insbesondere von 0,04 bis 1,1 Gew.%, jeweils bezogen auf das gesamte Mittel, enthalten, sind erfindungsgemäß bevorzugt.

Die Mund- und Zahnpflegemittel, insbesondere die Zahnpasten, können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Erfindungsgemäß bevorzugte Mund- und Zahnpflege- und -reinigungsmittel sind **dadurch gekennzeichnet, daß** sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 1,5 bis 5 Gew.-% und insbesondere von 1,75 bis 2,5 Gew.%, jeweils bezogen auf das gesamte Mittel, enthalten.

Die erfindungsgemäßen Mittel, insbesondere die Zahnpasten, können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Der Einsatz von Putzkörpern (Abrasiva) ist ebenfalls bevorzugt. Putzkörper sind amorphe, überwiegend anorganische, weitgehend wasserunlösliche, kleinstteilige Pulver, die keine scharfen Kanten aufweisen. Sie begünstigen in Zahn- und Mundpflegemitteln die Reinigung der Zähne und polieren gleichzeitig die Zahnoberfläche (Poliermittel).

Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident^{®} 12 DS (DEGUSSA) erhältlich.

Andere Fällungskieselsäuren dieser Art sind Sident^{®} 8 (DEGUSSA) und Sorbosil^{®} AC 39 (Crosfield Chemicals). Diese Kieselsäuren zeichnen sich durch eine geringere Verdickungswirkung und eine etwas höhere mittlere Teilchengröße von 8 --14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-kieselsäuren mit einer BET-Oberfläche von 150 -250 m²/g zu, z.B. die Handelsprodukte Sipernat 22 LS oder Sipernat^{®} 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die angegebenen Mengen beziehen sich auf die Gesamtmenge an Reibkörpern, wobei einzelne Reibkörper vorzugsweise in engeren Mengenbereichen eingesetzt werden. Erfindungsgemäß bevorzugte Mittel enthalten beispielsweise 5 bis 20 Gew.-%, vorzugsweise 8 bis 21 Gew.-%, weiter bevorzugt 9 bis 20 Gew.-% und insbesondere 11 bis 19 Gew.-% Kieselsäure(n). Weiter bevorzugte erfindungsgemäße Mittel sind **dadurch gekennzeichnet, daß** sie 0,25 bis 2 Gew.-%, vorzugsweise 0,5 bis 1,5 Gew.-% und insbesondere 0,75 bis 1,25 Gew.-% Aluminiumoxid enthalten.

Mund- und Zahnpflege- und reinigungsmittel, insbesondere Zahnpasten, können z.B. auch Substanzen enthalten, die gegen Plaque und/oder Zahnstein wirksam sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P ₂O₇, Na₂K₂P₂O₇, Na₂H₂P₂O₇ und K ₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind **dadurch gekennzeichnet, daß** sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Auch oberflächenaktive Substanzen sind in den erfindungsgemäßen Mitteln einsetzbar. Sie dienen beispielsweise in Zahnpasten zur Unterstützung der Reinigungswirkung und gewünschtenfalls auch zur Entwicklung von Schaum beim Zähnebürsten oder beim Mundspülen sowie zur Stabilisierung der Polierkörperdispersion im Träger und werden sowohl in Mundspüllösungen als auch in Zahnpasten üblicherweise in einer Menge von 0,1-5 Gew.-% eingesetzt.

Geeignete Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂- C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆ )-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z. B. Oxethylate von Fettsäuremono- und -diglyceriden, von Fettsäure-Sorbitanestern und Alkyl(oligo)-Glucoside.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten und Mundwässer oder Mundspüllösungen sind
- Oberflächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldi- glycol
- Pigmente, wie z.B. Titandioxid
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Es hat sich gezeigt, daß die erfindungsgemäßen Mund- und Zahnpflege- und -reinigungsmittel in ihrer Leistung weiter gesteigert werden können, wenn die Mittel salivationsfördernde Substanzen enthalten. Insbesondere die antibakterielle Wirkung und mit ihr die Antikarieswirkung und die Wirkung gegen Gingivitis und/oder Parodontitis werden hierdurch verstärkt.

Unter Salivation versteht man die Speichelproduktion und -freisetzung, im weiteren Sinne auch in unphysiologisch erhöhter Menge. Substanzen, die den Speichelfluß anregen und die Speichelmenge und/oder -freisetzung erhöhen, können aus den unterschiedlichsten Stoffklassen stammen.

Eine erfindungsgemäß beispielsweise geeignete Substanz ist das Pilocarpin, das in den erfindungsgemäßen Mund- und Zahnpflege- und -reinigungsmitteln enthalten sein kann.

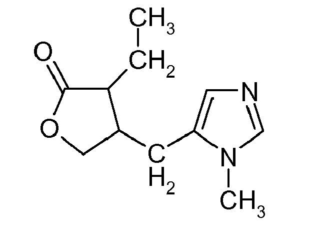

Weitere salivationsfördernde Substanzen sind insbesondere so genannte Scharfstoffe, d.h. scharf schmeckende und/oder ein Gefühl von Wärme erzeugende Substanzen. Erfindungsgemäß bevorzugte Mund- und Zahnpflege- und -reinigungsmittel sind **dadurch gekennzeichnet, daß** sie als salivationsfördernde Substanz mindestens eine scharf schmeckende und/oder ein Gefühl von Wärme erzeugende Substanz enthalten.

Als salivationsfördernden Inhaltsstoff enthalten die erfindungsgemäßen Erzeugnisse dieser Ausführungsform eine scharf schmeckende und/oder ein Gefühl von Wärme erzeugende Substanz. Diese Substanzen vermitteln dem Anwender einen scharfen, kribbelnden, mundwässernden oder wärmeerzeugenden Effekt, d.h. sie rufen sensorisch einen Wärmeeindruck oder ein Brennen, oder ein Prickeln, Perlen, Kitzeln oder Sprudeln hervor und fördern dadurch den Speichelfluß.

Erfindungsgemäß bevorzugte Erzeugnisse dieser Ausführungsform enthalten die scharf schmeckende(n) und/oder ein Gefühl von Wärme erzeugende(n) Substanz(en) in Mengen von 0,00001 bis 5 Gew.-%, vorzugsweise von 0,0005 bis 2,5 Gew.-%, weiter bevorzugt von 0,001 bis 1 Gew.-%, besonders bevorzugt von 0,005 bis 0,75 Gew.-% und insbesondere von 0,01 bis 0,5 Gew.-%, jeweils bezogen auf das Gewichts des gesamten Mittels.

Als scharf schmeckende oder ein Gefühl von Wärme erzeugende Substanz kann eine Reihe von Stoffen eingesetzt werden. Bevorzugt sind insbesondere N-Alkyl-substituierte Amide von ungesättigten Carbonsäuren, beispielsweise
- 2E,4E-Decadiensäure-N-Methylamid
- 2E,4E-Decadiensäure-N-Ethylamid
- 2E,4E-Decadiensäure-N-n-Propylamid
- 2E,4E-Decadiensäure-N-Isopropylamid
- 2E,4E-Decadiensäure-N-n-Butylamid
- 2E,4E-Decadiensäure-N-(1-Methylpropyl)-amid
- 2E,4E-Decadiensäure-N-Isobutylamid
- 2E,4E-Decadiensäure-N-tert-Butylamid
- 2E,4Z-Decadiensäure-N-Methylamid
- 2E,4Z-Decadiensäure-N-Ethylamid
- 2E,4Z-Decadiensäure-N-n-Propylamid
- 2E,4Z-Decadiensäure-N-Isopropylamid
- 2E,4Z-Decadiensäure-N-n-Butylamid
- 2E,4Z-Decadiensäure-N-(1-Methylpropyl)-amid
- 2E,4Z-Decadiensäure-N-Isobutylamid
- 2E,4Z-Decadiensäure-N-tert-Butylamid
- 2E,4E,8Z-Decatriensäure-N-Methylamid
- 2E,4E,8Z-Decatriensäure-N-Ethylamid
- 2E,4E,8Z-Decatriensäure-N-n-Propylamid
- 2E,4E,8Z-Decatriensäure-N-Isopropylamid
- 2E,4E,8Z-Decatriensäure-N-n-Butylamid
- 2E,4E,8Z-Decatriensäure-N-(1-Methylpropyl)-amid
- 2E,4E,8Z-Decatriensäure-N-Isobutylamid
- 2E,4E,8Z-Decatriensäure-N-tert-Butylamid
- 2E,4Z,8Z-Decatriensäure-N-Methylamid
- 2E,4Z,8Z-Decatriensäure-N-Ethylamid
- 2E,4Z,8Z-Decatriensäure-N-n-Propylamid
- 2E,4Z,8Z-Decatriensäure-N-Isopropylamid
- 2E,4Z,8Z-Decatriensäure-N-n-Butylamid
- 2E,4Z,8Z-Decatriensäure-N-(1-Methylpropyl)-amid
- 2E,4Z,8Z-Decatriensäure-N-Isobutylamid
- 2E,4Z,8Z-Decatriensäure-N-tert-Butylamid
- 2E,4E,8E-Decatriensäure-N-Methylamid
- 2E,4E,8E-Decatriensäure-N-Ethylamid
- 2E,4E,8E-Decatriensäure-N-n-Propylamid
- 2E,4E,8E-Decatriensäure-N-Isopropylamid
- 2E,4E,8E-Decatriensäure-N-n-Butylamid
- 2E,4E,8E-Decatriensäure-N-(1-Methylpropyl)-amid
- 2E,4E,8E-Decatriensäure-N-Isobutylamid
- 2E,4E,8E-Decatriensäure-N-tert-Butylamid
- 2E,4Z,8E-Decatriensäure-N-Methylamid
- 2E,4Z,8E-Decatriensäure-N-Ethylamid
- 2E,4Z,8E-Decatriensäure-N-n-Propylamid
- 2E,4Z,8E-Decatriensäure-N-Isopropylamid
- 2E,4Z,8E-Decatriensäure-N-n-Butylamid
- 2E,4Z,8E-Decatriensäure-N-(1-Methylpropyl)-amid
- 2E,4Z,8E-Decatriensäure-N-Isobutylamid
- 2E,4Z,8E-Decatriensäure-N-tert-Butylamid
- 2E,6Z,8E-Decatriensäure-N-Methylamid
- 2E,6Z,8E-Decatriensäure-N-Ethylamid
- 2E,6Z,8E-Decatriensäure-N-n-Propylamid
- 2E,6Z,8E-Decatriensäure-N-Isopropylamid
- 2E,6Z,8E-Decatriensäure-N-n-Butylamid
- 2E,6Z,8E-Decatriensäure-N-(1-Methylpropyl)-amid
- 2E,6Z,8E-Decatriensäure-N-Isobutylamid
- 2E,6Z,8E-Decatriensäure-N-tert-Butylamid
- 2E,6E,8E-Decatriensäure-N-Methylamid
- 2E,6E,8E-Decatriensäure-N-Ethylamid
- 2E,6E,8E-Decatriensäure-N-n-Propylamid
- 2E,6E,8E-Decatriensäure-N-Isopropylamid
- 2E,6E,8E-Decatriensäure-N-n-Butylamid
- 2E,6E,8E-Decatriensäure-N-(1-Methylpropyl)-amid
- 2E,6E,8E-Decatriensäure-N-Isobutylamid
- 2E,6E,8E-Decatriensäure-N-tert-Butylamid
- 2E,6Z,8Z-Decatriensäure-N-Methylamid
- 2E,6Z,8Z-Decatriensäure-N-Ethylamid
- 2E,6Z,8Z-Decatriensäure-N-n-Propylamid
- 2E,6Z,8Z-Decatriensäure-N-Isopropylamid
- 2E,6Z,8Z-Decatriensäure-N-n-Butylamid
- 2E,6Z,8Z-Decatriensäure-N-(1-Methylpropyl)-amid
- 2E,6Z,8Z-Decatriensäure-N-Isobutylamid
- 2E,6Z,8Z-Decatriensäure-N-tert-Butylamid
- 2E,6E,8Z-Decatriensäure-N-Methylamid
- 2E,6E,8Z-Decatriensäure-N-Ethylamid
- 2E,6E,8Z-Decatriensäure-N-n-Propylamid
- 2E,6E,8Z-Decatriensäure-N-Isopropylamid
- 2E,6E,8Z-Decatriensäure-N-n-Butylamid
- 2E,6E,8Z-Decatriensäure-N-(1-Methylpropyl)-amid
- 2E,6E,8Z-Decatriensäure-N-Isobutylamid
- 2E,6E,8Z-Decatriensäure-N-tert-Butylamid
- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid
- 2E,7E,9E-Undecatriensäure -N-Methylamid
- 2E,7E,9E-Undecatriensäure -N-Ethylamid
- 2E,7E,9E-Undecatriensäure -N-n-Propylamid
- 2E,7E,9E-Undecatriensäure -N-Isopropylamid
- 2E,7E,9E-Undecatriensäure -N-n-Butylamid
- 2E,7E,9E-Undecatriensäure -N-(1-Methylpropyl)-amid
- 2E,7E,9E-Undecatriensäure-N-isobutylamid
- 2E,7E,9E-Undecatriensäure -N-tert-Butylamid
- 2E,7Z,9Z-Undecatriensäure -N-Methylamid
- 2E,7Z,9Z-Undecatriensäure -N-Ethylamid
- 2E,7Z,9Z-Undecatriensäure -N-n-Propylamid
- 2E,7Z,9Z-Undecatriensäure -N-Isopropylamid
- 2E,7Z,9Z-Undecatriensäure -N-n-Butylamid
- 2E,7Z,9Z-Undecatriensäure -N-(1-Methylpropyl)-amid
- 2E,7Z,9Z-Undecatriensäure-N-isobutylamid
- 2E,7Z,9Z-Undecatriensäure -N-tert-Butylamid
- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid

Selbstverständlich sind auch andere Substitutionsmuster am Stickstoffatom möglich und bevorzugt, beispielsweise längerkettige n-Alkylreste (...-N-n-Pentylamid, ...-N-n-Pentylamid, ...-N-n-Pentylamid, ...-N-n-Pentylamid, ...-N-n-Pentylamid, ...-N-n-Hexylamid, ...-N-n-Heptylamid, ...-N-n-Octylamid, ...-N-n-Nonylamid, ...-N-n-Decylamid, ...-N-n-Undecylamid, ...-N-n-Dodecylamid, ...-N-n-Tridecylamid, usw.) oder disubstituierte ...-N,N-Dialkylamide wie ...-N,N-dimethylamid, ...-N,N-diethylamid, ...-N,N-di-n-propylamid, ...-N,N-diisopropylamid, ...-N,N-di-n-butylamid, ...-N,N-di(1-Methylpropyl)amid, ...-N,N-diisobutylamid, ...-N,N-di-tert-butylamid, ...-N,N-methyl-ethylamid, ...-N,N-methyl-n-propylamid, ...-N,N-methyl-isopropylamid, ...-N,N-ethyl-n-propylamid, ...-N,N-ethyl-isopropylamid, usw..

Unter den genannten Verbindungen sind einige im Rahmen der vorliegenden Erfindung besonders bevorzugt. Diese sind nachfolgend aufgeführt:
2E,6Z,8E-Decatriensäure-N-isobutylamid (N-Isobutyl-2E,6Z,8E-decatrienamid, auch Spilanthol oder Affinin genannt): 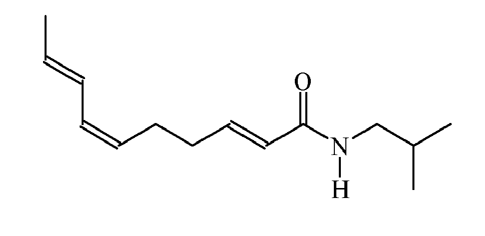
2E,4E,8Z-Decatriensäure-N-isobutylamid (N-Isobutyl-2E,4E,8Z-decatrienamid, auch Isoaffinin genannt): 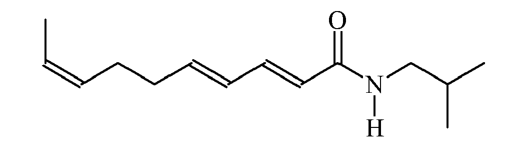
2E,7Z,9E-Undecatriensäure-N-isobutylamid (N-Isobutyl-2E,7Z,9E-undecatrienamid): 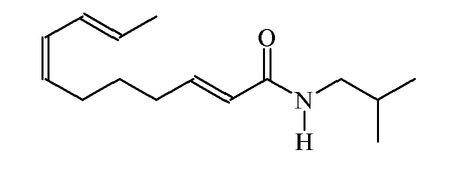
2E,4Z-Decadiensäure-N-isobutylamid (cis-Pellitorin): 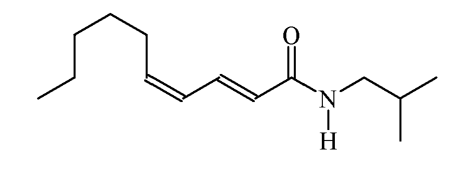
2E,4E-Decadiensäure-N-isobutylamid (trans-Pellitorin): 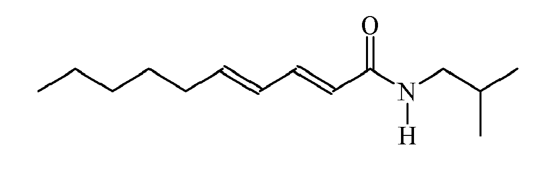 Ferulasäureamide, beispielsweise
   Ferulasäure-N-Vanillylamid: 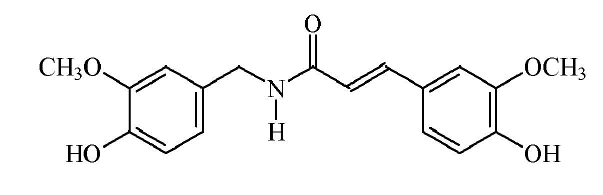
N-[2-(4-Hydroxy-3-methoxyphenyl)ethyl]-3-(4-hydroxy-3-methoxy-phenyl)- (2*E*)-propensäureamid (trans-Feruloylmethoxytyramin): 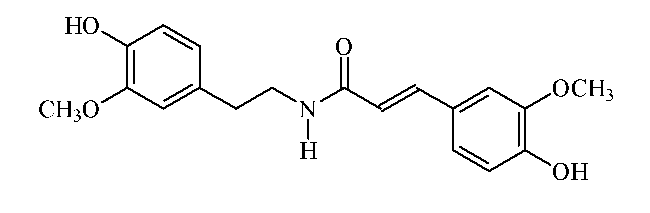
N-[2- (4-Hydroxy-3-methoxyphenyl)ethyl]-3-(4-hydroxy-3-methoxy-phenyl)-(2 Z)-propensäureamid (cis-Feruloylmethoxytyramin): 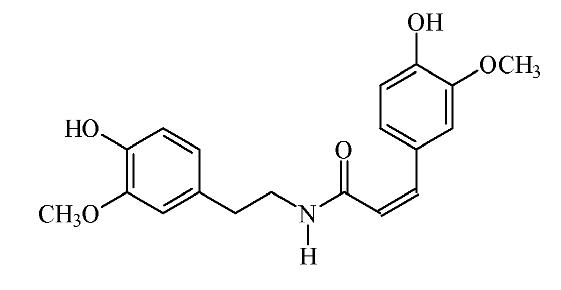
N-[2-(4- Hydroxy-3-methoxyphenyl)ethyl]-3-(4-hydroxy-3-methoxy-phenyl)- propansäureamid (Dihydroferuloylmethoxytyramin): 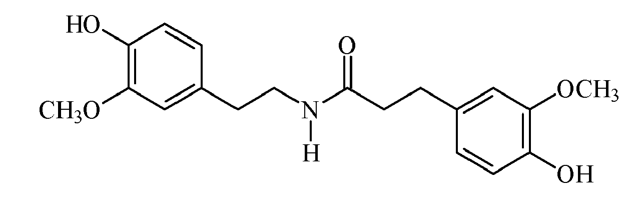
N-[2-(3,4- Dihydroxyphenyl)ethyl]-3-(4-hydroxy-3-methoxy-phenyl)-(2*E*)- propensäureamid (trans-Feruloyldopamin): 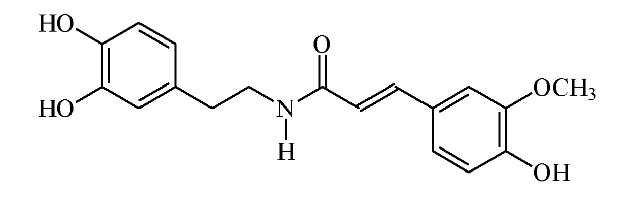
N-[2-(3,4-Dihydroxyphenyl) ethyl]-3-(4-hydroxy-3-methoxy-phenyl)-(2*Z*)-propensäureamid (cis-Feruloyldopamin): 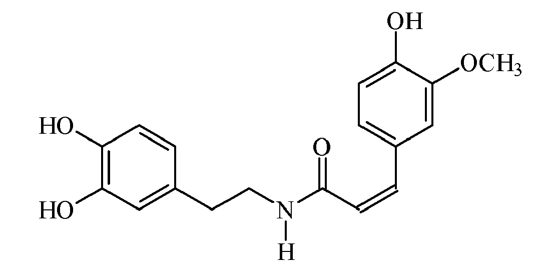
N-[2-(4-Hydroxyphenyl)ethyl]-3-(3,4- dihydroxyphenyl)-(2E)-propensäureamid (trans-Caffeoyltyramin): 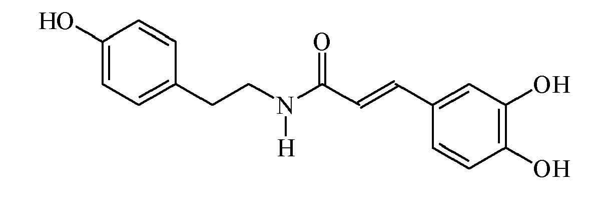
*N*-[2-(4-Hydroxyphenyl)ethyl]-3-(3,4-dihydroxyphenyl)-(2*Z*)- propensäureamid (cis-Caffeoyltyramin): 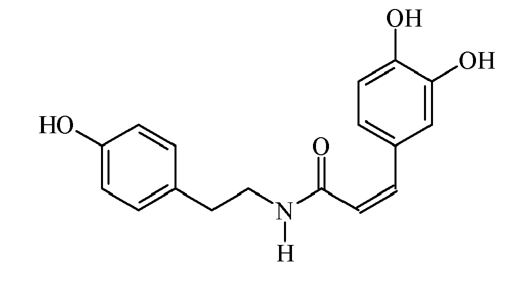
N-[2-(3,4-Dimethoxyphenyl) ethyl]-3-(3,4-dimethoxyphenyl)-(2*E*)-propensäureamid (trans-Rubenamin): 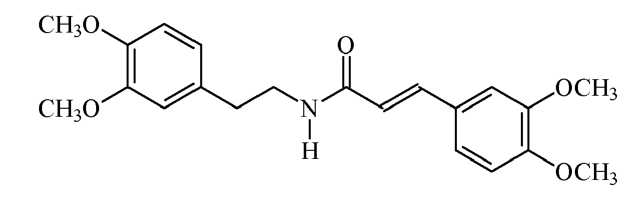
N-[2-(3,4-dimethoxyphenyl)ethyl]-3-(3,4- dimethoxy-phenyl)-(2*Z*)-propensäureamid (cis-Rubenamin):

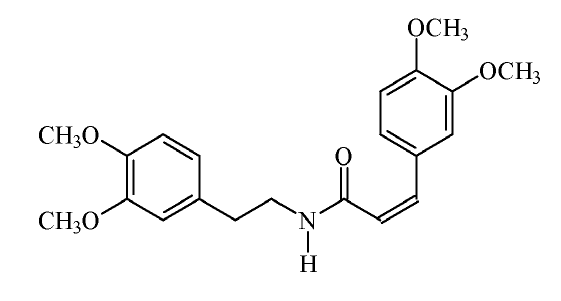

Weitere im Rahmen der vorliegenden Erfindung mit besonderem Vorzug einsetzbare Scharfstoffe sind beispielsweise Extrakte aus Naturpflanzen. Scharf schmeckende pflanzliche Extrakte können alle physiologisch unbedenklichen pflanzlichen Extrakte sein, die einen scharfen oder warmen sensorischen Eindruck hervorrufen. Bevorzugt als scharf schmeckende pflanzliche Extrakte sind beispielsweise Pfefferextrakt (*Piper ssp.,* insbesondere *Piper nigrum*)*,* Wasserpfefferextrakt (*Polygonum ssp.* ,insbesondere *Polygonum hydropiper*)*,* Extrakte aus *Allium* ssp.(insbesondere Zwiebel und Knoblauchextrakte), Extrakte aus Rettich (*Raphanus ssp.*), Meerrettichextrakte (*Cochlearia armoracia*), Extrakte aus schwarzem (*Brassica nigra*), wildem oder gelbem Senf (*Sinapis ssp.,* insbesondere *Sinapis arvensis* und *Sinapis alba*), Bertramwurzel-Extrakte (*Anacyclus ssp.,* insbesondere *Anacyclus pyrethrumL*.), Sonnenhutextrakte ( *Echinaceae ssp.*), Extrakten aus Szechuan-Pfeffer (*Zanthoxylum ssp.,* insbesondere *Zanthoxylum piperitum*), Spilanthesextrakt (*Spilanthes ssp.,* insbesondere *Spilanthes acmella*), Chiliextrakt (*Capsicum ssp.,* insbesondere *Capsicum frutescens* ), Paradieskörner-Extrakt ( *Aframomum* ssp.,insbesondere *Aframomum* melegueta[Rose] K. Schum.),Ingwerextrakt (*Zingiber* ssp.,insbesondere *Zingiber officinale*) und Galangaextrakt (*Kaempferia galanga oder Alpinia galanga*).

Eine besonders geeignete Substanz ist das aus dem Ingwerextrakt stammende Gingerol:

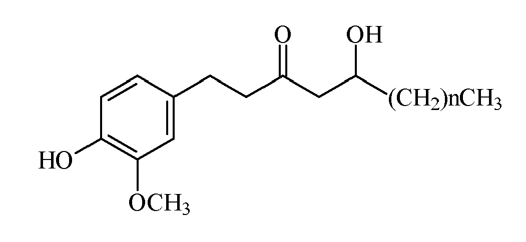

Einsetzbar ist auch N-Ethyl-p-menthan-3-carboxamid (N-Ethyl-5-Methyl-2-isopropylcyclohexancarboxamid):

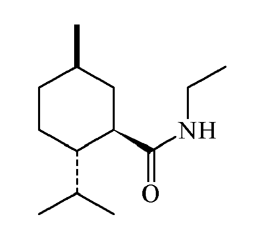

Andere scharf schmeckende oder ein Gefühl von Wärme erzeugende Substanzen können z.B. sein Capsaicin, Dihydrocapsaicin, Gingerol, Paradol, Shogaol, Piperin, Carbonsäure-N-vanillylamide, insbesondere Nonansäure-N- vanillylamid, 2-Alkensäureamide, insbesondere 2-Nonensäure-N-isobutylamid, 2- Nonensäure-N-4-hydroxy-3- methoxyphenylamid, Alkylether von 4-Hydroxy-3- methoxybenzylalkohol, insbesonders 4-Hydroxy-3-methoxybenzyl-n-butylether, Alkylether von 3- Hydroxy-4-methoxybenzylalkohol, Alkylether von 3,4- Dimethoxybenzylalkohol, Alkylether von 3-Ethoxy-4-hydroxybenzylalkohol, Alkylether von 3,4-Methylendioxybenzylalkohol, Nicotinaldehyd, Methylnicotinat, Propylnicotinat, 2-Butoxyethylnicotinat, Benzylnicotinat, 1-Acetoxychavicol, Polygodial oder Isodrimeninol.

Bevorzugte erfindungsgemäße remineralisierende Erzeugnisse sind **dadurch gekennzeichnet, daß** sie mindestens einen Scharfstoff aus der Gruppe der N-Alkyl-substituierte Amide von ungesättigten Carbonsäuren, vorzugsweise
a. 2E,6Z,8E-Decatriensäure-N-isobutylamid (Spilanthol) und/oder
b. 2E,4E,8Z-Decatriensäure-N-isobutylamid und/oder
c. 2E,7Z,9E-Undecatriensäure-N-isobutylamid und/oder
d. 2E,4Z-Decadiensäure-N-isobutylamid (cis-Pellitorin) und/oder
e. 2E,4E-Decadiensäure-N-isobutylamid (trans-Pellitorin) und/oder
f. Ferulasäure-N-Vanillylamid und/oder
g. N-[2-(4-Hydroxy-3-methoxyphenyl)ethyl]-3-(4-hydroxy-3-methoxy-phenyl)- (2*E*)-propensäureamid (trans-Feruloylmethoxytyramin) und/oder
h. N-[2- (4-Hydroxy-3-methoxyphenyl)ethyl]-3-(4-hydroxy-3-methoxy-phenyl)-(2 Z)-propensäureamid (cis-Feruloylmethoxytyramin) und/oder
i. N-[2-(4- Hydroxy-3-methoxyphenyl)ethyl]-3-(4-hydroxy-3-methoxy-phenyl)-propansäureamid (Dihydroferuloylmethoxytyramin) und/oder
j. N-[2-(3,4- Dihydroxyphenyl)ethyl]-3-(4-hydroxy-3-methoxy-phenyl)-(2E)-propensäureamid (trans-Feruloyldopamin) und/oder
k. N-[2-(3,4-Dihydroxyphenyl) ethyl]-3-(4-hydroxy-3-methoxy-phenyl)-(2*Z*)-propensäureamid (cis- Feruloyldopamin) und/oder
l. N-[2-(4-Hydroxyphenyl)ethyl]-3-(3,4- dihydroxyphenyl)-(2*E*)-propensäureamid (trans-Caffeoyltyramin) und/oder
m. *N*-[2-(4-Hydroxyphenyl)ethyl]-3-(3,4-dihydroxyphenyl)-(2*Z*)- propensäureamid (cis-Caffeoyltyramin) und/oder
n. N-[2-(3,4-Dimethoxyphenyl) ethyl]-3-(3,4-dimethoxyphenyl)-(2*E*)-propensäureamid (trans- Rubenamin) und/oder
o. N-[2-(3,4-dimethoxyphenyl)ethyl]-3-(3,4- dimethoxy-phenyl)-(2*Z*)-propensäureamid (cis-Rubenamin)
enthalten.

Zusätzlich zu den genannten Scharfstoffen oder an ihrer Stelle können auch weitere scharf schmeckende und/oder ein Gefühl von Wärme erzeugende Substanzen in die erfindungsgemäßen Erzeugnisse eingearbeitet werden.

Als besonders geeignet haben sich im Rahmen der vorliegenden Erfindung alkylsubstituierte Dioxane der Formel 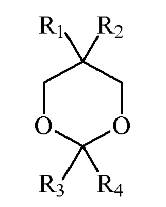 erwiesen, in der R1 und R2 unabhängig voneinander ausgewählt sind aus -H, -CH₃, -CH₂CH₃ und R3 und R4 unabhängig voneinander ausgewählt sind aus -H, -CH₃, -CH₂CH₃, -CH₂CH₂CH₃, - CH(CH₃)₂.

Als weiterhin besonders geeignet haben sich im Rahmen der vorliegenden Erfindung Phenylester der Formel 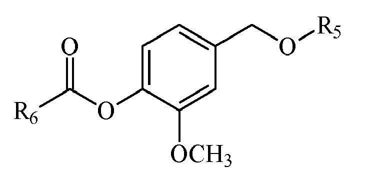 erwiesen, in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht

Als weiterhin besonders geeignet haben sich im Rahmen der vorliegenden Erfindung Carvonacetale der Formel 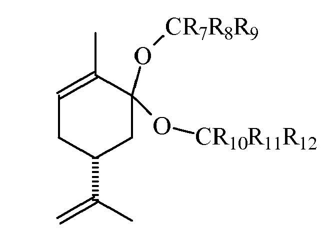 erwiesen, in der R7 bis R12 unabhängig voneinander ausgewählt sind aus -H, -CH₃, - CH₂CH₃, -CH₂CH₂CH₃, -CH(CH₃)₂, -CH₂ CH₂CH₂CH₃, -CH₂CH(CH₃)₂, -CH(CH₃)CHzCH₃, -C(CH₃)₃ oder R9 und R10 zusammen eine chemische Bindung oder eine Gruppe -(CR13R14)ₓ bedeuten, worin x für die Werte 1 oder 2 steht und R13 und R14 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, - CH(CH₃)CH₂CH₃, C(CH₃)₃

Durch den Einsatz der Silberionen wird die antibakterielle Wirkung antibakterieller Substanzen erhöht. Insbesondere für pflanzliche antibakterielle Wirkstoffe, die in der Regel eine schwächere und weniger lang anhaltende Wirkung aufweisen, ist der zusätzliche Einsatz von Silberionen hilfreich. Diese Substanzen zeigen in in-vitro-Versuchen eine Unterstützung der Suppression der Entzündungsreaktion und eine Steigerung der Zellaktivität und somit potentiell eine Steigerung des Zellwachstums, was wiederum den Heilungsprozeß der durch die Entzündungsreaktion verursachten Schäden unterstützt. Der Einsatz von Silberionen führt zu einem schnelleren Abklingen des Entzündungsprozesses und zu einer besseren Regeneration der Mundschleimhaut.

Ein weiterer Gegenstand der vorliegenden Erfindung ist daher die Verwendung von Silberionen zur Steigerung der antibakteriellen Wirkung antibakterieller Verbindungen.

Erfindungsgemäße Mittel können als Zahnpasten oder Zahncremes formuliert werden. Ein weiterer Gegenstand der vorliegenden Erfindung ist ein Verfahren zur Vorbeugung vor und Behandlung von Karies und/oder Bekämpfung von Halitosis und/oder zur Behandlung von Gingivitis oder Parodontitis, bei dem eine erfindungsgemäße Zubereitung auf eine Zahnbürste aufgetragen und mit dieser die Zähne geputzt werden.

Die erfindungsgemäßen Zusammensetzungen lassen sich auch als Mundspüllösungen oder Mundwässer formulieren. Ein weiterer Gegenstand der vorliegenden Erfindung ist ein Verfahren zur Vorbeugung vor und Behandlung von Karies und/oder Bekämpfung von Halitosis und/oder zur Behandlung von Gingivitis oder Parodontitis, bei dem eine erfindungsgemäße Zubereitung in Form einer Mundspüllösung in die Mundhöhle eingebracht und dort für einen Zeitraum von mindestens 10 Sekunden, vorzugsweise mindestens 20 Sekunden und insbesondere mindestens 45 Sekunden belassen wird.

Bezüglich bevorzugter Ausführungsformen der erfindungsgemäßen Verwendungen und der erfindungsgemäßen Verfahren gilt mutatis mutandis das zu den erfindungsgemäßen Mitteln Gesagte.

Beispiele (alle Angaben in Gew.-%):

| **Mundwasser-Formulierungen** | **I** | **II** |
|---|---|---|
| Cocoamidopropylbetain, 30%ig in Wasser | - | 0,86 |
| C₁₂₋₁₄-Alkylpolyglycosid | | |
| (Oligomerisierungsgrad = 1,4), 50%ig in Wasser | 0,1 | - |
| Sorbitol 70% | 3,0 | 3,0 |
| Natriumfluorid | 0,045 | 0,045 |
| Saccharin Natrium | 0,03 | 0,03 |
| Fettsäureamidoalkylbetain (30% Aktivsubstanz) | 0,4 | 0,4 |
| Thymol | 0,08 | 0,08 |
| Silbercitrat | 0,0007 | 0,0007 |
| Aroma | 0,1 | 0,1 |
| Wasser, vollentsalzt | ad 100 | ad 100 |

| **Zahnpasta-Formulierung** | | |
|---|---|---|
| Sorbitol 70% | 60,0 | |
| Fällungskieselsäure (Sident^{®} 8) | 10,0 | |
| Fällungskieselsäure (Sident^{®} 22 S) | 8,5 | |
| Dinatriumphosphat, wasserfrei | 0,1 | |
| Trinatriumphosphat, wasserfrei | 0,2 | |
| Natriumfluorid | 0,32 | |
| PEG 1500 | 1,5 | |
| Titandioxid | 0,5 | |
| Natriumlaurylsulfat | 1,5 | |
| Saccharin Natrium | 1,0 | |
| Xanthan | 0,4 | |
| Aroma | 1,2 | |
| Thymol | 0,08 | |
| Silbercitrat | 0,0007 | |
| Wasser, vollentsalzt | ad 100 | |

## Patentansprüche

1. Mund- und Zahnpflege- und -reinigungsmittel, enthaltend - bezogen auf sein Gewicht -
a) 0,1 bis 100 ppm Silberionen,
b) 0,001 bis 5 Gew.-% mindestens einer antibakteriellen Verbindung.

2. Mund- und Zahnpflege- und -reinigungsmittel nach Anspruch 1, **dadurch gekennzeichnet, daß** es - bezogen auf sein Gewicht - 0,2 bis 75 ppm, vorzugsweise 0,5 - 50 ppm, besonders bevorzugt 0,75 - 25 ppm und insbesondere 1 bis 10 ppm Silberionen enthält.

3. Mund- und Zahnpflege- und reinigungsmittel nach einem der Ansprüche 1 oder 2, **dadurch gekennzeichnet, daß** es mindestens ein Silbersalz aus der Gruppe Silbercitrat, Silberlactat, Silberacetat, Silbersulfat, Silbermala, Silbersuccinat, Silbertartrat, Silbermandelat, Silbersalicylat, Silbergluconat enthält.

4. Mund- und Zahnpflege- und -reinigungsmittel nach einem der Ansprüche 1 bis 3, **dadurch gekennzeichnet, daß** es - bezogen auf sein Gewicht - 0,15 bis 150 ppm (1,5 10⁻⁶ bis 0,0015 Gew.-%), vorzugsweise 0,25 bis 125 ppm (2,5 10⁻⁶ bis 0,00125 Gew.-%), besonders bevorzugt 0,5 bis 100 ppm (5 10⁻⁶ bis 0,001 Gew.-%) und insbesondere 1 bis 90 ppm (1 10⁻⁵ bis 0,0009 Gew.-%) Silbercitrat enthält.

5. Mund und Zahnpflege- und -reinigungsmittel nach einem der Ansprüche 1 bis 4, **dadurch gekennzeichnet, daß** es als Silbersalz ausschließlich Silbercitrat enthält.

6. Mund- und Zahnpflege- und -reinigungsmittel nach einem der Ansprüche 1 bis 5, **dadurch gekennzeichnet, daß** es - bezogen auf sein Gewicht - 0,0025 bis 2,5 Gew.-%, vorzugsweise 0,005 bis 1 Gew.-%, besonders bevorzugt 0,01 bis 0,5 Gew.-% und insbesondere 0,025 bis 0,1 Gew.-% antibakterielle(n) Wirkstoff(e) enthält.

7. Mund- und Zahnpflege- und -reinigungsmittel nach einem der Ansprüche 1 bis 6, **dadurch gekennzeichnet, daß** der bzw. die antibakteriellen Wirkstoffe ausgewählt ist/sind aus p-Hydroxybenzoesäuremethyl-, -ethyl- oder propylester und/oder Bromchlorophen und/oder Triclosan und/oder Phenyl-Salicylsäureester und/oder Biguaniden, vorzugsweise Chlorhexidin.

8. Mund- und Zahnpflege- und -reinigungsmittel nach einem der Ansprüche 1 bis 7, **dadurch gekennzeichnet, daß** der bzw. die antibakteriellen Wirkstoffe ausgewählt ist/sind aus Thymol und/oder Eugenol und/oder Eucalyptol und/oder Extrakten von Myrrhe und/oder Extrakten von Salbei und/oder Extrakten von Nelke.

9. Mund und Zahnpflege- und -reinigungsmittel nach einem der Ansprüche 1 bis 8, **dadurch gekennzeichnet, daß** es - bezogen auf sein Gewicht - 0,5 bis 60 Gew.-%, vorzugsweise 0,75 bis 55 Gew.-%, besonders bevorzugt 1 bis 50 Gew.-% und insbesondere 2 bis 40 Gew.-% mindestens eines mehrwertigen Alkohols aus der Gruppe Sorbit und/oder Glycerin und/oder 1,2-Propylenglycol.-% oder deren Mischungen enthält.

10. Mund- und Zahnpflege- und -reinigungsmittel nach einem der Ansprüche 1 bis 9, **dadurch gekennzeichnet, daß** es zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,02 bis 2,5 Gew.% und insbesondere von 0,04 bis 1,1 Gew.%, jeweils bezogen auf das gesamte Mittel, enthält.

11. Mund- und Zahnpflege- und -reinigungsmittel nach einem der Ansprüche 1 bis 10, **dadurch gekennzeichnet, daß** es die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 1,5 bis 5 Gew.-% und insbesondere von 1,75 bis 2,5 Gew.%, jeweils bezogen auf das gesamte Mittel, enthält.

12. Mund- und Zahnpflege- und -reinigungsmittel gemäß einem der Ansprüche 1 bis 11, **dadurch gekennzeichnet, daß** es zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ -2H₂O), Natriumaluminiumsilikate, insbesondere Zeolith A, organische Polymere, insbesondere Polymethacrylate oder Gemische dieser Reibkörper,vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthält.

13. Verwendung von Silberionen zur Steigerung der antibakteriellen Wirkung antibakterieller Verbindungen.

14. Verfahren zur Vorbeugung vor und Behandlung von Karies und/oder Bekämpfung von Halitosis und/oder zur Behandlung von Gingivitis oder Parodontitis, **dadurch gekennzeichnet, daß** eine Zubereitung nach einem der Ansprüche 1 bis 12 auf eine Zahnbürste aufgetragen und mit dieser die Zähne geputzt werden.

15. Verfahren zur Vorbeugung vor und Behandlung von Karies und/oder Bekämpfung von Halitosis und/oder zur Behandlung von Gingivitis oder Parodontitis, **dadurch gekennzeichnet, daß** eine Zubereitung nach einem der Ansprüche 1 bis 12 in Form einer Mundspüllösung in die Mundhöhle eingebracht und dort für einen Zeitraum von mindestens 10 Sekunden, vorzugsweise mindestens 20 Sekunden und insbesondere mindestens 45 Sekunden belassen wird.
